# EUROPEAN PATENT APPLICATION

(11) **EP 1 308 431 A2**
(43) Date of publication of application: **07.05.2003**
(21) Application number: 02253279.0
(22) Date of filing: 10.05.2002
(51) Int. Cl.: C07C 29/149, C07C 31/20, B01J 23/72

(54) **Process for preparing 1,3-alkandiols from 3-hydroxyester**

(30) Priority: 01.11.2001 KR 2001067901
(71) Applicant: SAMSUNG ELECTRONICS CO., LTD., Suwon-City, Kyungki-do 442-742 (KR); Korea Research Institute of Chemical Technology, Daejun-si 305-390 (KR)
(72) Inventor: Lee, Byeong No, Joongrang-Gu, Seoul 131-222 (KR); Jung, In Sun, Paldal-Gu, Suwong-Shi, 442-813 (KR); Jang, Eun Joo, Yusung-Gu, Daejun-Shi 305-390 (KR); Lee, Jung Ho, Yunsung-Gu, Daejun-Shi 305-333 (KR); Kim, Hyung Rok, Yusung-Gu, Daejun-Shi 305-340 (KR); Han, Yo Han, Daejun-Shi 305-333 (KR)
(74) Representative: Kyle, Diana

(57) **Abstract**

A process for preparing a 1,3-alkandiol from a 3-hydroxyester, comprising the steps of: i) preparing a catalyst through adding an alkaline precipitator to an aqueous copper salt solution to form copper hydroxide particles and ageing the particles following the addition of a colloidal silica thereto; ii) activating the catalyst through reduction with H₂ gas or H₂/N₂ mixed gas applying a pressure of 5 to 2000 psig at a temperature of 100 to 250 °C in the presence of an activation solvent; and iii) hydrogenating a 3-hydroxyester in a liquid phase slurry manner with H₂ gas or H₂/N₂ mixed gas applying a pressure of 50 to 3000 psig at a temperature of 100 to 250 °C in the presence of the activated catalyst and a reaction solvent is disclosed. By virtue of the present invention, a 1,3-alkanediol can be selectively prepared from a 3-hydroxyester in a high yield.

## Description

The present invention relates, to a process for preparing a 1,3-alkandiol from a 3-hydroxyester, particularly to a process for preparing a 1,3-alkandiol from a 3-hydroxyester in a liquid phase slurry manner with high yield and selectivity.

1,3-Alkandiols have been widely used as coating materials and intermediates for various organic syntheses, as well as raw materials for the production of polyesters. At present, several processes are known for preparing 1,3-alkandiols. For example, processes for preparing a 1,3-alkandiol by hydroformylating an epoxide into a 3-hydroxyaldehyde derivative and then hydrogenating the 3-hydroxyaldehyde derivative have been proposed (US Patent Nos. 5,770,776, 5,723,389, 5,731,478 and 5,777,182). Alternatively, processes for preparing a 1,3-alkandiol by hydrating acrolein into 3-hydroxypropanal, followed by hydrogenation of the 3-hydroxypropanal, have also been proposed (US Patent Nos. 6,232,511 and 6,140,543). Another method has been reported to provide 1,3-alkanediols through a certain biological reaction, wherein glycerol is used as a starting material (US Patent Nos. 6,136,576, 6,013,494 and 5,821,092). Commercially, Shell Co. (Louisiana, USA) have succeeded in producing 1,3-propandiol through the hydrogenation of 3-hydroxypropanal resulting from the hydroformylation of ethylene oxide. However, the processes in which 3-hydroxyaldehyde or derivatives thereof, such as 3-hydroxypropanal, are generated as intermediates are disadvantageous in that these intermediates are so unstable as to be likely to oligomerize or to be converted into other side products including acetals. Thus, hydrogenation thereof into corresponding 1,3-alkanediols is often incomplete and consequently the quality of the final product is deteriorated.

Even though an alternative process was suggested, wherein a 1,3-alkandiol is prepared by carboesterifying an epoxide with carbon monoxide and an alcohol to afford a 3-hydroxyester followed by hydrogenation of the ester group of the 3-hydroxyester, this process has not been put to practical use in the industrial field. This is due to the fact that the reaction pathway is very unselective for 1,3-alkandiols when a conventional hydrogenation catalyst, such as copper-chromium oxide, copper-zinc oxide or Raney nickel is used. Typically, hydrogenation of hydroxyester compounds having an hydroxyl group at a non-β position, which is involved in, for example, synthesis of 1,4-butanediol or ethylene glycol, can be easily accomplished with high selectivity and yield, because side products resulting from dehydration are hardly produced. On the other hand, β-hydroxyester compounds including 3-hydroxyesters are likely to undergo a dehydration reaction resulting in undesired side products.

While a number of Cu- or noble metal-containing catalysts have been studied and developed for use in preparing alcohols from their corresponding carbonyl group-containing compounds, particularly from esters, few catalytic processes have been reported that are useful for the preparation of 1,3-alkandiols from 3-hydroxyesters having a hydroxyl group at the specific β-position. WO 00/18712 and US Patent No. 6,191,321 disclose the use of a Cu/ZnO-based catalyst in the preparation of 1,3-propandiol in the presence of an alcohol solvent such as methanol. While the alcohol solvent is helpful to suppress the self-lactonization and degradation of the reactant, 3-hydroxyesters, an alcohol with low boiling point is unfavorable because it cannot maintain high selectivity at a high conversion rate and prolonged reaction stability of the catalyst under H₂ gas flow in a fixed-bed catalyst reactor. Moreover, the reactant, 3-hydroxyester, is similar to the product, 1,3-alkandiol, in terms of chemical properties and boiling point, making it difficult to isolate and purify the product from the reactant in the case of a low conversion rate.

Meanwhile, we have proposed a process for preparing a 1,3-alkandiol from a 3-hydroxyester in the gas phase or liquid-gas phase, by the use of a CuO/SiO₂-based catalyst shaped into nanoparticles with a diameter of 4 to 10 nm (Korean Patent Appln. No. 2000-71643). According to this process, 1,3-alkandiols can be obtained with relatively high selectivity owing to high catalytic activity. However, the process is unsatisfactory for increasing the selectivity for 1,3-alkandiols over 90%, and the generation of lactone and other side products with high boiling points is likely to occur when the concentration of the reactant is high. Furthermore, stability of the catalyst may be affected by possible degradation occurring during the course of reduction of the catalyst.

A feature of the present invention is to solve the above-mentioned problems arising in the prior art and to provide a novel process for preparing a 1,3-alkandiol from a 3-hydroxyester whereby the increase of the selectivity for 1,3-alkanediols to 90% or more, as well as the prolongation of the stability of the catalyst, can be accomplished.

According to the present invention, there is provided a novel process for preparing a 1,3-alkandiol from a 3-hydroxyester, comprising the steps of:
i) preparing a catalyst through adding an alkaline precipitator to an aqueous copper salt solution to form copper hydroxide particles and ageing the particles following the addition of a colloidal silica thereto;
ii) activating the catalyst through reduction with H₂ gas or H₂/N₂ mixed gas applying a pressure of 5 to 2000 psig at a temperature of 100 to 250°C in the presence of an activation solvent; and
iii) hydrogenating a 3-hydroxyester in a liquid phase slurry manner with H₂ gas or H₂/N₂ mixed gas applying a pressure of 50 to 3000 psig at a temperature of 100 to 250°C in the presence of the activated catalyst and a reaction solvent.

According to the present invention, Cu-containing catalysts stabilized with silica are applied to a hydrogenation process for preparing a 1,3-alkandiol from a 3-hydroxyester. The major component of the catalysts is Cu in the form of oxide, and the weight ratio of copper oxide (CuO) to silica (SiO₂) in the catalyst is 95:5 to 50:50. The silica-stabilized copper oxide catalysts can be generally represented by CuO-SiO₂. However, the conventional CuO-SiO₂ catalysts prepared by impregnating a conventional silica carrier with copper oxide, for example, as described in US Patent No. 4,511,744 is inadequate to the hydrogenation process of the present invention. To secure the high catalytic activity required in the present invention, the CuO-SiO₂ catalyst should be prepared in a particular manner of the present invention wherein micro-particles (diameter ≤ 10nm) of the copper oxide precursor are stabilized with silica. That is, the catalyst used in the present invention is prepared by adding an alkaline precipitator, such as an alkali metal carbonate or sodium hydroxide, to an aqueous copper salt solution, and then ageing the resulting copper hydroxide particles in the presence of a colloidal silica. Significantly, the silica constituting the catalyst of the present invention functions as an essential component, and thus it is clearly discriminated from the conventional carriers.

The copper salt used in the present invention can be exemplified by copper nitrate, copper chloride, copper acetate and copper sulfate, and the preferred concentration thereof in the aqueous copper salt solution is 5 to 25 wt%.

The colloidal silica used in the present invention can be made by any of the known methods (See: for example, "The Chemistry of Silica", John Wiley & Sons, Inc., New York, 1979, pp. 331 to 334). Also, commercially available products such as Ludox AS (DuPont, 30 to 40 wt% of silica), Snowtex (Nissan Chemical Industry, 30 to 40 wt% of silica), SILIFOG (Acehitech, 30 to 40 wt% of silica) can be used.

According to the present invention, the hydrogenation catalyst may further comprise one or more promoters in order to improve its hydrogenation activity or selectivity. Preferred promoters can be exemplified by Re, Ru, Pd, Pt, Rh, Ag, Se, Te, Mo and Mn, and their content in the catalyst is preferably 0.001 to 10 mol%, and more preferably 0.003 to 7 mol% based on Cu.

The catalyst of the present invention can be formed by any of the known methods including the conventional extruding method, pelleting method, and impregnating method using thermal-resistant carriers. The catalyst thus formed is then calcined at 200 to 800°C, preferably 300 to 700°C, for 2 to 10 hrs.

The calcined catalyst is in the form of oxide, and therefore it should be activated prior to use, preferably by reduction with hydrogen or hydrogen-containing gas at 100 to 250°C for 1 to 60 hrs. In general, this activation step is performed while flowing the hydrogen or hydrogen-containing gas diluted with nitrogen or argon gas into a reactor filled with the calcined oxide catalyst in the presence of an appropriate organic solvent. There are, however, apprehensions about catalyst degradation by heat generated in the course of reduction. The present invention aims to prevent the catalyst from being degraded during reduction, as well as from being converted into an undesired form, by reducing the catalyst in the presence of a specific activation solvent, based on that the following hydrogenation step is carried out in a liquid phase slurry manner. Thus, the activation of the catalyst according to the present invention can be achieved with greater efficiency than that of the conventional liquid-gas phase or gas phase method. In the activation of the catalyst, the pressure of the hydrogen or hydrogen-containing gas is preferably maintained between 5 to 2000 psig.

Preferred examples of the activation solvent include high boiling point solvents such as tetraethylene glycol dimethyl ether (hereinafter, referred to as "TEGDME"), pentaethylene glycol dimethyl ether and sulfolane, as well as low boiling point solvents such as pentane, hexane, 1,4-dioxane and methanol.

The activation solvent may be removed by filtration and/or washing prior to the following hydrogenation of 3-hydroxyesters, or alternatively, may be mixed with a reaction solvent used in the hydrogenation reaction without further treatment. Where the activation solvent and the reaction solvent are mixed together, the mixing ratio of the former to the latter is preferably between 5:95 and 90:10 (w/w).

According to the present invention, the hydrogenation catalyst may be used after modification with an alkylsilane compound in order to improve its catalytic activity and selectivity. At this time, hydroxyl groups of silica in the catalyst are masked with the alkylsilane compound, whereby the hydrophilic catalyst changes into hydrophobic. As the alkylsilane compound, trialkoxymonoalkylsilane, dialkoxydialkylsilane or monoalkoxytrialkylsilane can be used, wherein the alkyl group consists of 1 to 30 carbon atoms, and the alkoxy group is a C₁₋₅ linear or non-linear alkoxy group, preferably a methoxy or ethoxy group. Such alkylsilane compounds can be exemplified by trimethoxypropylsilane, trimethoxyoctylsilane, dimethoxydimethylsilane, dimethoxymethylpropylsilane, dimethoxymethyloctylsilane, methoxytrimethylsilane, methoxydimethylpropylsilane and methoxydimethyloctylsilane.

3-hydroxyesters used as a substrate in the hydrogenation reaction according to the present invention can be represented by the following formula (I): 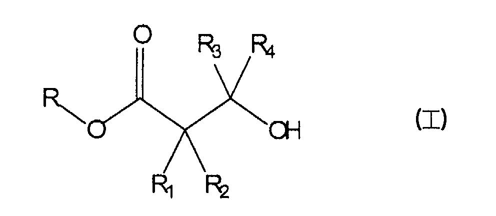 in the above formula (I),
wherein each of R₁, R₂, R₃, R₄ and R, independently, is a hydrogen atom, a C₁₋₂₀ non-branched saturated aliphatic hydrocarbon, branched saturated aliphatic hydrocarbon, saturated cyclic hydrocarbon or ring-containing aliphatic hydrocarbon, or a hydrocarbon derived from the substitution of ester, hydroxyl and/or alkoxy groups for hydrogen atoms at one or more carbon chains of any of the aforementioned C₁₋₂₀ hydrocarbon species.

In the above formula (I), R of the ester group is preferably methyl, ethyl, n-propyl, isopropyl, n-butyl, isobutyl, t-butyl, cyclohexyl, or cyclohexane methyl.

Preferred examples of the 3-hydroxyesters include methyl(or ethyl) 3-hydroxypropionate, 3-hydroxybutyric ester, 3-hydroxypentanoic ester, 3-hydroxyheptanoic ester, 3-hydroxyoctanoic ester, 3-hydroxynonanoic ester, 3-hydroxydecanoic ester, 2-methyl-3-hydroxypropanoic ester, 2-methyl-3-hydroxybutanoic ester, 2-methyl-3-hydroxypentanoic ester, 2-methyl-3-hydroxyhexanoic ester, 2-methyl-3-hydroxyheptanoic ester, 2-methyl-3-hydroxyoctanoic ester, 2-methyl-3-hydroxynonanoic ester, 2-methyl-3-hydroxydecanoic ester, 2-ethyl-3-hydroxybutanoic ester, 2-ethyl-3-hydroxypentanoic ester, 2-ethyl-3-hydroxyhexanoic ester, 2-ethyl-3-hydroxyheptanoic ester, 2-ethyl-3-hydroxyoctanoic ester, 2-ethyl-3-hydroxynonanoic ester, and 2-ethyl-3-hydroxydecanoic ester.

The process for preparing 1,3-alkandiols from 3-hydroxyesters by using the hydrogenation catalyst according to the present invention is characterized in that hydrogenation of 3-hydroxyesters is carried out in a liquid phase slurry manner. In the liquid phase slurry method according to the present invention, the catalyst is not immobilized to form a layer unlike in the conventional gas phase and liquid-gas phase methods. Instead, the catalyst of the present invention exists in the form of slurry and consequently can readily contact with hydrogen and the substrate dissolved in a reaction solvent to catalyze the hydrogenation reaction vigorously. The hydrogenation reaction according to this liquid phase slurry method of the present invention provides advantages unexpected from the conventional gas phase and liquid-gas phase methods. Namely, there is a limit to increase the selectivity for 1,3-alkanediols below 90% by the conventional gas phase and liquid-gas phase methods, whereas the present invention can easily achieve high selectivity for 1,3-alkanediols above 90%, preferably amounting to 100%, by controlling overall reaction conditions including concentration of the reactant and pressure of the hydrogen gas. To produce 1,3-alkanediols in a high yield, high selectivity for 1,3-alkanediols should be secured notwithstanding the conversion rate being low. This is because if selectivity for 1,3-alkanediols is high, in spite of a low conversion rate, then unreacted, i.e., remnant 3-hydroxyesters, can be separated and recycled to the hydrogenation reaction, and accordingly efficiency of the use of the reactant significantly increases. On the other hand, although the conversion rate is high, low selectivity for 1,3-alkanediols results in side products rather than desired 1,3-alkanediols, whereby overall reaction efficiency decreases.

Further, by use of the liquid phase slurry method of the present invention, both conversion rate and selectivity can be highly improved while using much less hydrogen than in the conventional liquid-gas phase or gas phase method. Moreover, the conventional liquid-gas phase and gas phase methods have been known to have disadvantages in that lactone is inevitably generated at a high concentration of the reactant, whereas the liquid phase slurry method of the present invention produces little lactone, and so cumbersome separation of lactone and environmental pollution caused by toxic side products generated therefrom can be avoided.

According to the present invention, 3-hydroxyesters are supplied to the hydrogenation reaction as dissolved in an alcohol solvent or in a mixed solvent consisting of an alcohol and a high-boiling point solvent that boils at a higher temperature than the reactant and product for the following purposes of: (i) suppressing side reactions including lactonization and condensation reactions between the reactant, 3-hydroxyester, as well as between the reactant and the product, (ii) enhancing catalytic activity and selectivity of the catalyst by controlling concentration of the reactant to contact the catalyst during the reaction, and (iii) relieving the significant decrease of selectivity of the catalyst at a high conversion rate. Thus, 3-hydroxyesters are dissolved in the alcohol or mixed solvent to a concentration of 2 to 98 wt%.

The alcohol solvent used in the hydrogenation reaction according to the present invention is not specifically limited, but C₁₋₅ linear or non-linear alcohols, such as methanol, ethanol, propanol, isopropanol, n-butanol, isobutanol, t-butanol, etc, are preferred, allowing easy separation and purification of the reaction product from the solvent.

As the high-boiling point solvent used in conjunction with the alcohol solvent, any solvent can be used, provided that it can be readily mixed with 3-hydroxyesters and has a boiling point higher than that of 1,3-alkandiols so that it can be readily separated therefrom. Preferably, ethers such as TEGDME, pentaethylene glycol dimethyl ether and sulfolane, are used. The mixing ratio of the alcohol to the high boiling point solvent is preferably between 5:95 and 90:10 (w/w).

In the hydrogenation reaction according to the present invention, the reaction temperature is adjusted in the range of 100 to 250°C, preferably 120 to 200°C, and the reaction pressure used is in the range of 50 to 3,000 psig, preferably 150 to 2,000 psig.

The present invention can be more clearly understood with reference to the following examples. It should be understood that the following examples are not intended to restrict the scope of the present invention in any manner.

### Example 1

To a solution containing 60.0g of [Cu(NO₃)₂•3H₂O] and 2.54g of [Mn(NO₃)₂•6H₂O] in 400 ml of distilled water was added 129 ml of aqueous NaOH solution (16 wt%) to form coprecipitates. The coprecipitates were allowed to age at 70 to 80°C for 4 hrs, following addition of 12.8 g of aqueous colloidal silica, Ludox AS-40 (ammonium-stabilized type, 40 wt% of silica), to the solution. The resulting slurry was recovered by filtration, followed by washing with distilled water drying at 120 °C for 12 hrs. Then, the dry powder was shaped by pressing and crushed again into particles having 20 to 40 mesh size. The particles were calcined at 450°C under an atmosphere for 6 hrs to afford a CuO(77wt%)-SiO₂(20wt%)-MnO₂(3wt%) catalyst.

### Example 2

To a 600ml high pressure reactor was sequentially added 5.0 g of the CuO(77wt%)-SiO₂(20wt%)-MnO₂(3wt%) catalyst obtained from the above Example 1 and 250 ml of tetraethylene glycol dimethyl ether (TEGDME) as an activation solvent. Subsequently, 5% H₂/N₂ mixed gas was flowed into the reactor to a pressure of 500 psig, and the temperature in the reactor was elevated to 180°C, where reduction of the catalyst was continued for 20 hrs, to afford activated catalyst.

The activated catalyst was recovered and washed several times with methanol under nitrogen gas atmosphere. To 5.0 g of the washed catalyst was then added 5.0 g of 3-hydroxymethyl propionate (HPM) and 350 ml of methanol as a reaction solvent, and a hydrogenation reaction was carried out for 20 hrs at 150°C under a pressure of 1450 psig. The resulting product was collected into a sampling tube and subjected to GC analysis. The result is set forth in Table 1.

### Example 3

The CuO(77wt%)-SiO₂(20wt%)-MnO₂(3wt%) catalyst obtained from Example 1 was activated through reduction according to the same manner as in Example 2, except that the reduction was continued for 2 hrs.

The activated catalyst was recovered and washed several times with methanol under a nitrogen gas atmosphere. To 10.0 g of the washed catalyst was then added 10.0 g of HPM and 300 ml of methanol as a reaction solvent, and hydrogenation reaction was carried out for 15 hrs at 150°C under a pressure of 1450 psig. The resulting product was collected into a sampling tube and subjected to GC analysis. The result is set forth in Table 1.

### Example 4

The CuO(77wt%)-SiO₂(20wt%)-MnO₂(3wt%) catalyst obtained from Example 1 was activated through reduction according to the same manner as in Example 2, except that the reduction was continued for 44 hrs.

The activated catalyst was recovered and washed several times with methanol under nitrogen gas atmosphere. To 10.0 g of the washed catalyst was then added 10.0 g of HPM and 250 ml of methanol as a reaction solvent, and hydrogenation reaction was carried out for 20 hrs at 150°C under a pressure of 1450 psig. The resulting product was collected into a sampling tube and subjected to GC analysis. The result is set forth in Table 1.

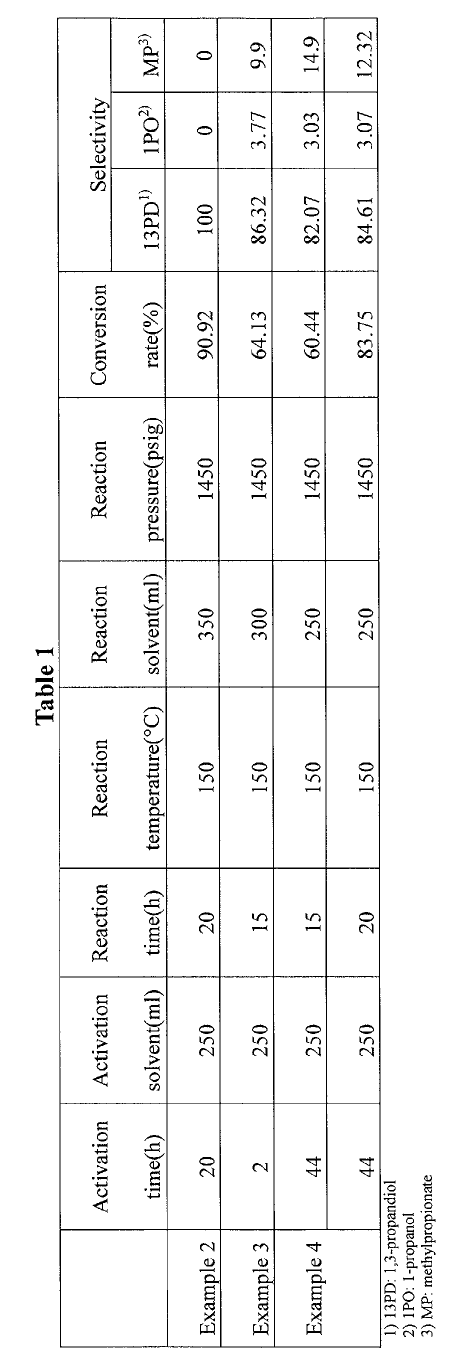

### Example 5

To a 600 ml high pressure reactor was sequentially added 5.0 g of the CuO(77wt%)-SiO₂(20wt%)-MnO₂(3wt%) catalyst obtained from the above Example 1 and 250 ml of tetraethylene glycol dimethyl ether (TEGDME) as an activation solvent. Subsequently, 5% H₂/N₂ mixed gas was flowed into the reactor to a pressure of 500 psig, and the temperature in the reactor was elevated to 180°C, where reduction of the catalyst was continued for 20 hrs, to afford activated catalyst.

The activated catalyst was recovered and washed several times with methanol under nitrogen gas atmosphere. To 5.0 g of the washed catalyst was then added 5.0 g of 3-hydroxymethyl propionate (HPM) and 350 ml of methanol as a reaction solvent, and a hydrogenation reaction was carried out for 4 hrs at 150°C under a pressure of 1450 psig. The resulting product was collected into a sampling tube and subjected to GC analysis. The result is set forth in Table 2.

### Example 6

To a 600 ml high pressure reactor was sequentially added 10.0 g of the CuO(77wt%)-SiO₂(20wt%)-MnO₂(3wt%) catalyst obtained from the above Example 1 and 250 ml of tetraethylene glycol dimethyl ether(TEGDME) as an activation solvent. Subsequently, 5% H₂/N₂ mixed gas was flowed into the reactor to a pressure of 500 psig, and temperature in the reactor was elevated to 180°C, where reduction of the catalyst was continued for 2 hrs, to afford activated catalyst.

The activated catalyst was recovered and washed several times with methanol under nitrogen gas atmosphere. To 10.0 g of the washed catalyst was then added 5.0 g of 3-hydroxymethyl propionate (HPM) and 300 ml of methanol as a reaction solvent, and a hydrogenation reaction was carried out for 2 hrs at 150°C under a pressure of 1450 psig. The resulting product was collected into a sampling tube and subjected to GC analysis. The result is set forth in Table 2.

### Example 7

The procedure of Example 7 was conducted according to the same manner as in Example 6, except that the reduction of the catalyst was continued for 44 hrs and the hydrogenation reaction for 3 hrs while using 250 ml of methanol as the reaction solvent. The resulting product was collected into a sampling tube and subjected to GC analysis. The result is set forth in Table 2.

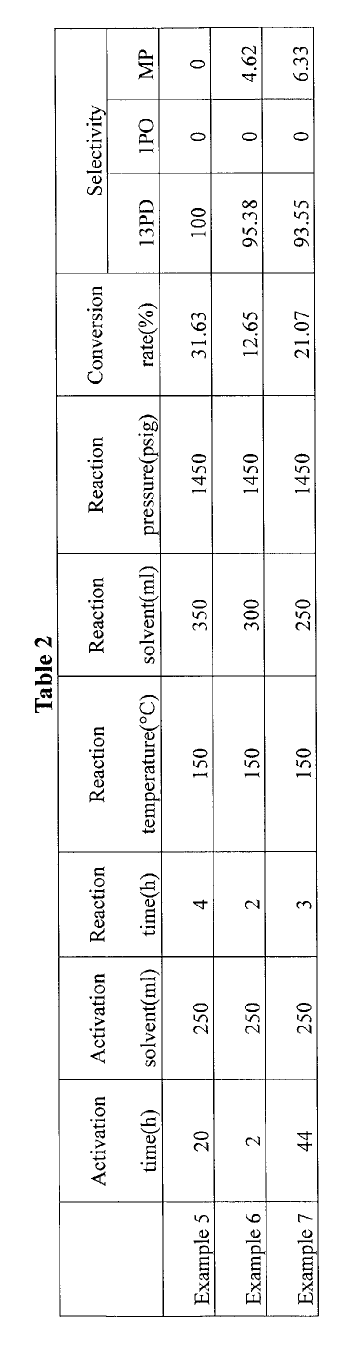

### Example 8

To a 600 ml high pressure reactor was sequentially added 10.0 g of the CuO(77wt%)-SiO₂(20wt%)-MnO₂(3wt%) catalyst obtained from Example 1 and 250 ml of tetraethylene glycol dimethyl ether (TEGDME) as an activation solvent. Subsequently, H₂ gas was flowed into the reactor to a pressure of 1000 psig, and the temperature in the reactor was elevated to 180°C, where reduction of the catalyst was continued for 4 hrs, to afford activated catalyst.

Without washing, to the activated catalyst in TEGDME was added 10.0 g of 3-hydroxymethyl propionate (HPM) and 200 ml of methanol as a reaction solvent, and hydrogenation reaction was carried out for 16 hrs at 165°C under a pressure of 1450 psig. The resulting product was collected into a sampling tube and subjected to GC analysis. The result is set forth in Table 3.

### Example 9

The procedure of Example 9 was conducted according to the same manner as in Example 8, except that the hydrogenation reaction was carried out at 150°C. The resulting product was collected into a sampling tube and subjected to GC analysis. The result is set forth in Table 3.

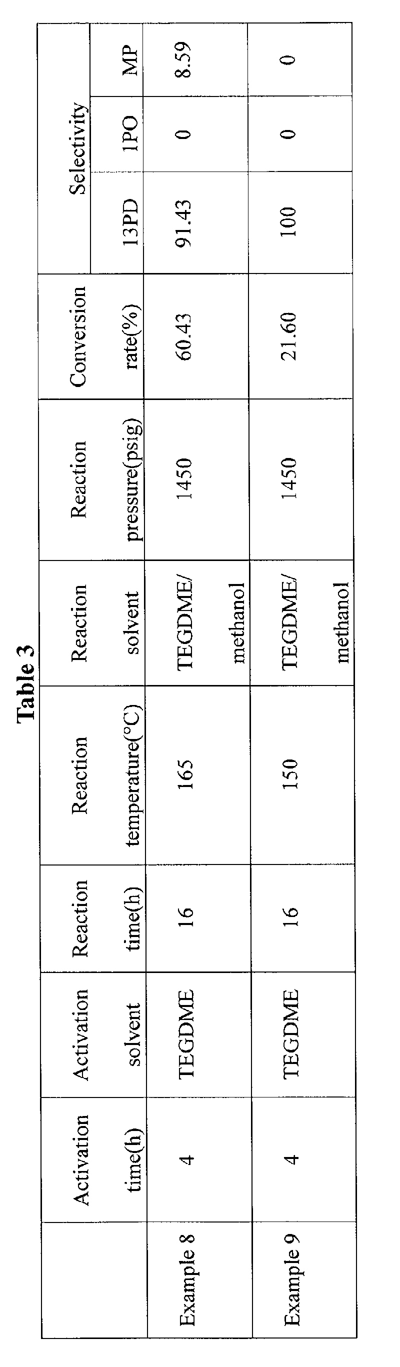

### Example 10

To a 600 ml high pressure reactor was sequentially added 20.0 g of the CuO(77wt%)-SiO₂(20wt%)-MnO₂(3wt%) catalyst obtained from the above Example 1 and 200 ml of tetraethylene glycol dimethyl ether (TEGDME) as an activation solvent. Subsequently, H₂ gas was flowed into the reactor to a pressure of 1000 psig, and the temperature in the reactor was elevated to 180°C, where reduction of the catalyst was continued for 40 hrs to afford activated catalyst.

Without washing, to the activated catalyst in TEGDME was added 10.0 g of 3-hydroxymethyl propionate(HPM) and 200 ml of methanol as a reaction solvent, and hydrogenation reaction was carried out at 165°C under a pressure of 1450 psig, while increasing the reaction time up to 18 hrs. The resulting product was collected into a sampling tube and subjected to GC analysis. The result is set forth in Table 4. Meanwhile, the catalyst was recovered from the reaction mixture, followed by washing with methanol and air-drying for further use.

### Example 11

Using the catalyst recovered from Example 10, the hydrogenation reaction was conducted according to the same manner as in Example 10. The resulting product was collected into a sampling tube and subjected to GC analysis. The result is set forth in Table 4.

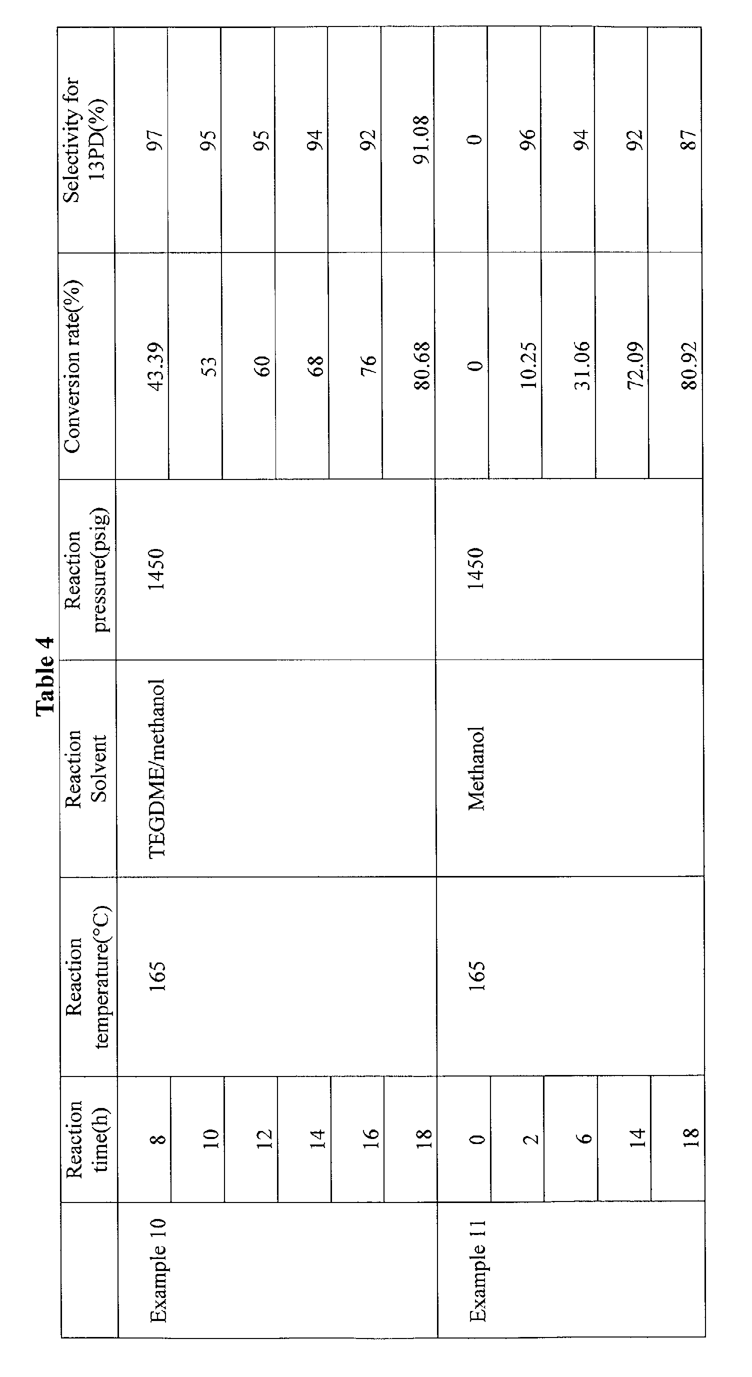

As stated above, by virtue of the present invention, it is possible to selectively prepare a 1,3-alkanediol from a 3-hydroxyester in a high yield.

## Claims

1. A process for preparing a 1,3-alkandiol from a 3-hydroxyester, comprising the steps of:
i) preparing a catalyst through adding an alkaline precipitator to an aqueous copper salt solution to form copper hydroxide particles and ageing the particles following the addition of a colloidal silica thereto;
ii) activating the catalyst through reduction with H₂ gas or H₂/N₂ mixed gas applying a pressure of 5 to 2000 psig at a temperature of 100 to 250°C in the presence of an activation solvent; and
iii) hydrogenating a 3-hydroxyester in a liquid phase slurry manner with H₂ gas or H₂/N₂ mixed gas applying a pressure of 50 to 3000 psig at a temperature of 100 to 250°C in the presence of the activated catalyst and a reaction solvent.

2. The process according to the claim 1, wherein the alkaline precipitator is an alkali metal carbonate or sodium hydroxide.

3. The process according to claim 1 or 2, wherein the aqueous copper salt solution is an aqueous 5 to 25 wt% solution of copper nitrate, copper chloride, copper acetate or copper sulfate.

4. The process according to any one of claims 1 to 3, wherein the major component of the catalyst is copper in the form of oxide and the weight ratio of CuO to SiO₂ in the catalyst is between 95:5 and 50:50.

5. The process according to any one of claims 1 to 4, wherein the catalyst further comprises one or more promoters selected from Re, Pd, Ru, Pt, Rh, Ag, Se, Te, Mo and Mn in an amount of 0.001 to 10 mol% based on Cu.

6. The process according to any one of claims 1 to 5, wherein the 3-hydroxyester is represented by the formula (I): 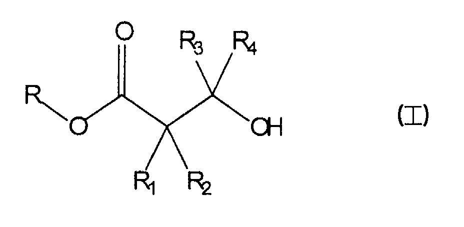 in the above formula (I),
wherein each of R₁, R₂, R₃, R₄ and R, independently, is a hydrogen atom, a C₁₋₂₀ non-branched saturated aliphatic hydrocarbon, branched saturated aliphatic hydrocarbon, saturated cyclic hydrocarbon or ring-containing aliphatic hydrocarbon, or a hydrocarbon derived from the substitution of ester, hydroxyl and/or alkoxy groups for hydrogen atoms at one or more carbon chains of any of said C₁₋₂₀ hydrocarbon species.

7. The process according to claim 6, wherein R is methyl, ethyl, n-propyl, isopropyl, n-butyl, isobutyl, t-butyl, cyclohexyl, or cyclohexane methyl.

8. The process according to any one of claims 1 to 7, wherein the activation solvent is a high boiling point solvent selected from tetraethylene glycol dimethyl ether, pentaethylene glycol dimethyl ether and sulfolane.

9. The process according to any one of claims 1 to 7, wherein the activation solvent is a low boiling point solvent selected from pentane, hexane, 1,4-dioxane and methanol.

10. The process according to any one of claims 1 to 9, wherein the mixing ratio of the activation solvent to the reaction solvent is between 5:95 and 90:10(w/w).

11. The process according to any one of claims 1 to 10, wherein the step (iii) is carried out without removing the activation solvent from the catalyst activated in step (ii).

12. The process according to any one of claims 1 to 10, wherein the step (iii) is carried out after removing the activation solvent from the catalyst activated in the step (ii).

13. The process according to any one of claims 1 to 12, wherein the reaction solvent used in the step (iii) is an alcohol solvent, or a mixed solvent consisting of an alcohol and a high-boiling point solvent that boils at a higher temperature than 1,3-alkandiol.

14. The process according to claim 13, wherein the alcohol solvent is selected from methanol, ethanol, propanol, isopropanol, n-butanol, isobutanol, and t-butanol.

15. The process according to claim 13 or 14 wherein the high-boiling point solvent is tetraethylene glycol dimethyl ether, pentaethylene glycoldimethyl ether or sulfolane, and the mixing ratio of the alcohol solvent to the high-boiling point solvent is between 5:95 and 90:10(w/w).

16. The process according to any one of claims 1 to 15, further comprising the step (iv) of recovering remnant 3-hydroxyester from the hydrogenation reaction mixture at the end of the step (iii) and repeating the step (iii) by using the recovered 3-hydroxyester.
